(19) Europäisches Patentamt
European Patent Office
Office européen des brevets

(11) Publication number: **0 099 676 B1**

(12) # EUROPEAN PATENT SPECIFICATION

(45) Date of publication of patent specification: **22.04.87**

(51) Int. Cl.⁴: **C 07 C 43/04**, C 07 C 41/09

(21) Application number: **83303798.9**

(22) Date of filing: **30.06.83**

(54) **Catalytic preparation of dimethyl ether.**

(30) Priority: **01.07.82 US 394120**

(43) Date of publication of application:
**01.02.84 Bulletin 84/05**

(45) Publication of the grant of the patent:
**22.04.87 Bulletin 87/17**

(84) Designated Contracting States:
**AT BE CH DE FR GB IT LI LU NL SE**

(56) References cited:
**DE-C- 844 740**
**US-A-4 042 633**

**Patent Abstracts of Japan Vol. 4, no. 159, 6 November 1980 Page 30 C 30**

**Patent Abstracts of Japan Vol. 2, no. 52, 14 April 1978 Page 289 C 78**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Brake, Loren Dale**
**3324 Rockfield Drive South**
**Wilmington Delaware 19810 (US)**

(74) Representative: **Woodcraft, David Charles et al**
**BROOKES & MARTIN High Holborn House 52/54 High Holborn**
**London, WC1V 6SE (GB)**

(56) References cited:

**Chemical Abstracts vol. 65, no. 2, 18 July 1966, Columbus, Ohio, USA W.H. WADE et al. "Dehydration of methyl alcohol and isopropyl alcohol on aluminas of various specific surface areas", column 1443 b-e**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 099 676 B1

**Description**

This invention relates to an improved method for the preparation of dimethyl ether by the catalytic dehydration of methanol. It is more particularly directed to such a method in which the catalyst used is an alumino-silicate containing a high proportion of alumina.

Dimethyl ether is a commodity in the chemical industry, widely used as a starting material in the preparation of other chemicals, *e.g.,* dimethyl sulfate, and more recently, as a propellant in aerosol containers.

One of the commonly used methods for preparing dimethyl ether is the catalytic dehydration of methanol, using a phosphoric acid-alumina catalyst.

While that process is generally satisfactory, the catalyst has a tendency to coke, which makes it necessary to replace it more frequently than is desirable. By "coke" is meant the phenomenon by the which the surface of the catalyst becomes coated with carbon, thus blocking its pores and reducing its effectiveness.

It has now been found that this coking can be minimized if, instead of the phosphoric acid-alumina catalyst, one uses aluminosilicate catalyst containing 1—20 wt% silica and 80—99 wt% alumina. Surprisingly, use of such a catalyst not only reduces the amount of coking but also significantly increases the rate of dehydration reaction over that obtained with the phosphoric acid-alumina catalyst, and greatly reduces the number and amounts of by products formed, notably hydrogen, carbon monoxide, methane, ethane, propane, ethylene, propylene and various ethers having high boiling points.

Chemical abstracts, Vol 65, No. 2, 18 July 1966, Columbus, Ohio, USA, W. H. Wade et al, "Dehydration of methyl alcohol and isopropyl alcohol on aluminas of very specific surface areas" discloses the dehydration of methanol on $Al_2O_3$ catalyst at 180—370 degrees C, dimethyl ether and water being the main reaction products. This art does not, however, refer to the use of alumina in combination with silica and, in the result, performance compared to such a mixed catalyst is lost.

J. Colloid Interface Sci., Vol. 21, No. 4, 1966, Pages 349—357 shows a further example of the large variety of alumina-containing catalysts which have been used or proposed in production of organic materials over a large number of years. This art discloses the use of kaolin to effect etherification of ethylene glycol with a lower alkanol such as methanol, temperatures of 200—300°C, being employed. In order to obtain commercially acceptable yields, it is believed that a temperature of 275—400°C, would be necessary and at these temperatures the coking problems of high silica content catalysts would be evident, resulting in short use of the catalyst.

Accordingly, the invention provides a process for the preparation of dimethyl ether which comprises the vapour phase dehydration of methanol using an aluminosilicate catalyst and conducting the reaction at a temperature of 200 to 500°C and at a pressure from ambient to 500 psi (3447 Kpa) gauge characterised in that the aluminosilicate contains by weight 1 to 20% of silica and 80 to 99% of alumina.

The catalytic dehydration of methanol to form dimethyl ether is well known and is described in detail in U.S. Patent 2,014,408.

The reaction proceeds according to the general equation:—

$$2CH_3OH \rightleftharpoons CH_3OCH_3 + H_2O$$

The product of the reaction is principally a mixture of dimethyl ether, unreacted methanol and water.

The reaction is ordinarily conducted continuously in a column reactor in the vapor phase, generally at a maximum bed temperature of 200—500°C, preferably 275—420°C, and normally at a pressure ranging from ambient to 3447 Kpa (gauge), preferably 1034—1723 Kpa (gauge). The catalyst is in practice packed into the reactor in the customary way, and the vaporized and preheated (200—300°) methanol passed through it, preferably downwardly. Residence time of the methanol in the reactor is determined according to well-known chemical engineering principles, as are the methods of recovering the dimethyl ether from the reactor effluent and the methods of refining it.

The catalysts used according to the invention are aluminosilicates containing 1—20% by weight of silica and 80—99% by weight of alumina, preferably 1—10% of silica and 90—99% of alumina, even more preferably about 6% of silica and about 94% of alumina. A catalyst especially suited for use according to the invention is an aluminosilicate containing about 6% silica and about 94% alumina, sold by Harshaw Chemical Company of Beachwood, Ohio, as A1—1602.

The catalysts can be made by the well-known method of coprecipitating appropriate amounts of sodium silicate and sodium aluminate from aqueous solution by bringing the solution to a pH of about 8 with hydrochloric acid, at a temperature of 50—70°C. The resulting gel, a mixture of hydroxides, is recovered, then normally washed free of chlorides, dried to a free-flowing powder, pelleted and then calcined at 450—650°C to give a material which can be used directly as the catalyst.

The catalyst is customarily used in the form of cylindrical pellets. Pellet size is selected according to recognized chemical engineering principles, and is usually in the range 2—130 mm in all dimensions. The pore volume, pore size and total surface area of the pellets are likewise a matter of choice, and will generally be in the ranges of 0.2—0.8 cc/g, greater than 25 angstrom units, and 100—250 $m^2/g$, respectively.

Example—best mode

In the following description, all parts are by weight.

Six hundred parts of Harshaw A1—1602 in the form of 6.5 mm cylindrical pellets were packed into an adiabatic column reactor having a length/diameter ratio of 5.

Methanol, preheated to 270°C, was then continuously fed into the top of the reactor. The vapors were passed downwardly through the catalyst bed at a rate of 9000 parts per hour. Pressure in the reactor was 1034 kPa (gauge) and the reactor bed temperature reached a maximum of 390°C.

The vapors leaving the reactor were condensed to give a product having the average composition:—

| | |
|---|---|
| Dimethyl ether | 57% |
| Methanol | 20% |
| Water | 23% |

**Claims**

1. A process for the preparation of dimethyl ether which comprises the vapour phase dehydration of methanol using an aluminosilicate catalyst and conducting the reaction at a temperature of 200 to 500°C and at a pressure from ambient to 500 psi (3447 Kpa gauge) characterised in that the aluminosilicate contains by weight 1 to 20% of silica and 80 to 99% of alumina.

2. A process as claimed in claim 1 in which the catalyst contains 1 to 10% by weight of silica and 90 to 99% by weight of alumina.

3. A process as claimed in claim 1 or claim 2 in which the catalyst contains about 6% by weight of silica and about 94% by weight of alumina.

4. A process as claimed in any one of the preceding claims in which the catalyst is formed by co-precipitating a mixture of hydroxides of silicon and aluminium from aqueous solution of sodium silicate and sodium aluminate by bringing the solution to a pH of about 8 with hydrochloric acid at a temperature of 50° to 70°C, recovering the resulting mixture of hydroxides, washing the mixture free of chlorides and drying to a free-flowing powder, pelleting the powder, and calcining the pellets at 450° to 650°C.

5. A process as claimed in any one of the preceding claims in which the catalyst has a pore volume in the range of 0.2 to 0.8 cc/g, a pore size greater than 25 Angstrom units, and a surface area of 100 to 250 $m^2/g$.

6. A process as claimed in any one of the preceding claims in which the methanol is contacted with the catalyst at a temperature of from 275°C to 420°C.

7. A process as claimed in any one of the preceding claims in which the methanol is contacted with the catalyst at a pressure of from 150 psig (1034 Kpa gauge) to 250 psig (1723 Kpa gauge).

8. A process according to any one of the preceding claims which is operated continuously by passing preheated methanol through a reactor column packed with the catalyst.

**Patentansprüche**

1. Verfahren zur Herstellung von Dimethylether, bei dem Methanol unter Verwendung eines Alumosilikat-Katalysators in der Dampfphase dehydratisiert und die Reaktion bei einer Temperatur von 200 bis 500°C und bei einem Druck von Umgebungsdruck bis zu 500 psi (3447 Kpa gauge) durchgeführt wird, dadurch gekennzeichnet, dass das Alumosilikat 1 bis 20 Gew.% Siliciumdioxid und 80 bis 99 Gew.% Aluminiumoxid enthält.

2. Verfahren nach Anspruch 1, bei dem der Katalysator 1 bis 10 Gew.% Siliciumdioxid und 90 bis 99 Gew.% Aluminiumoxid enthält.

3. Verfahren nach Anspruch 1 oder 2, bei dem der Katalysator etwa 6 Gew.% Siliciumdioxid und etwa 94 Gew.% Aluminiumoxid enthält.

4. Verfahren nach einem der vorhergehenden Ansprüche, bei dem der Katalysator durch gemeinsame Ausfällung eines Gemisches von Hydroxiden von Silicium und Aluminium aus einer wässrigen Lösung von Natriumsilikat und Natriumaluminat gebildet wird, indem man die Lösung mit Salzsäure bei einer Temperatur von 50 bis 70°C auf einen pH-Wert von etwa 8 bringt, das entstandene Gemisch der Hydroxide abtrennt, es frei von Chloriden wäscht und zu einem frei fliessenden Pulver trocknet, das Pulver pelletiert und die Pellets bei 450 bis 650°C calciniert.

5. Verfahren nach einem der vorhergehenden Ansprüche, bei dem der Katalysator ein Porenvolumen im Bereich von 0,2 bis 0,8 cc/g, eine Porengrösse von mehr als 25 Angström und eine Oberfläche von 100 bis 250 $m^2/g$ hat.

6. Verfahren nach einem der vorhergehenden Ansprüche, bei dem das Methanol mit dem Katalysator bei einer Temperatur von 275 bis 420°C kontaktiert wird.

7. Verfahren nach einem der vorhergehenden Ansprüche, bei dem das Methanol mit dem Katalysator bei einem Druck von 150 psig (1034 Kpa gauge) bis 250 psig (1723 Kpa gauge) kontaktiert wird.

8. Verfahren nach einem der vorhergehenden Ansprüche, welches kontinuierlich durchgeführt wird, indem man vorerhitztes Methanol durch eine mit dem Katalysator gepackte Reaktorkolonne leitet.

**Revendications**

1. Un procédé pour la préparation d'éther diméthylique qui comprend la déhydratation de méthanol en phase vapeur avec utilisation d'un catalyseur aluminosilicate et la conduite de la réaction à une température de 200 à 500°C et à une pression allant de la pression ambiante à environ 3447 kPa (500 psi) au manomètre, caractérisé en ce que l'aluminosilicate contient en poids 1 à 20% de silice de 80 à 99% d'alumine.

2. Un procédé selon la revendication 1, dans

lequel le catalyseur contient 1 à 10% en poids de silice et 90 à 99% en poids d'alumine.

3. Un procédé selon la revendication 1 ou 2, dans lequel le catalyseur contient environ 6% en poids de silice et environ 94% en poids d'alumine.

4. Un procédé selon l'une quelconque des revendications précédentes, dans laquel on forme le catalyseur par coprécipitation d'un mélange d'hydroxyde de silicium et d'aluminium à partir d'une solution aqueuse de silicate de sodium et d'aluminate de sodium, en portant la solution à un pH d'environ 8 avec de l'acide chlorhydrique à une température de 50 à 70°C, en récupérant le mélange d'hydroxydes obtenu, en débarrassant le mélange de chlorures par lavage et en le séchant en une poudre s'écoulant librement, en pastillant la poudre, et en calcinant les pastilles à 450 à 650°C.

5. Un procédé selon l'une quelconque des revendications précédentes, dans lequel le catalyseur a un volume de pores dans la gamme de 0,2 à 0,8 $cm^3/g$, une grosseur de pores supérieure à 25 Å, et une surface spécifique de 100 à 250 $m^2/g$.

6. Un procédé selon l'une quelconque des revendications précédentes, dans lequel on met le méthanol en contact avec le catalyseur à une température de 275 à 420°C.

7. Un procédé selon l'une quelconque des revendications précédentes, dans lequel on met le méthanol en contact avec le catalyseur à une pression de 1034 kPa au manomètre (150 psig) à 1723 kPa au manomètre (250 psig).

8. Un procédé selon l'une quelconque des revendications précédentes, que l'on met en oeuvre de façon continue en faisant passer du méthanol préchauffé à travers une colonne de réacteur garnie de catalyseur.